# EUROPEAN PATENT SPECIFICATION

(11) **EP 1 458 419 B1**
(45) Date of publication and mention of the grant of the patent: **04.05.2005**
(21) Application number: 02792971.0
(22) Date of filing: 11.12.2002
(51) Int. Cl.: A61L 12/08, A45C 11/00, A45C 11/04, A01N 25/34, A61L 2/26

(54) **ANTIMICROBIAL CONTACT LENS CASE**
ANTIMIKROBIELLER KONTAKTLINSENBEHÄLTER
ETUI ANTIMICROBIEN POUR LENTILLES DE CONTACT

(30) Priority: 12.12.2001 US 340930 P
(43) Date of publication of application: 22.09.2004
(73) Proprietor: Novartis AG, 4056 Basel (CH); Novartis Pharma GmbH, 1230 Wien (AT)
(72) Inventor: LINDLEY, Karen, Frances, Cumming, GA 30041 (US); GILLIARD, Allen, Buford, GA 30519 (US); TAPALE, Mayuresh, Shashikant, Atlanta, GA 30316 (US)
(86) International application number: PCT/EP2002/014085
(87) International publication number: WO 2003/049776

(56) References cited:
- WO-A-00/14128
- WO-A-00/38552
- WO-A-95/34327
- WO-A-99/52362
- US-A- 5 515 117

## Description

### FIELD OF THE INVENTION

The invention relates generally to antimicrobial contact lens cases, and more particularly to lens cases having antimicrobial compounds or chemicals embedded in the body of the lens case.

### BACKGROUND OF THE INVENTION

Containers for storing contact lenses are known in various designs. Usually such containers are filled with a disinfecting liquid and for their storage, in particular for overnight storage, the contact lenses are put into the liquid and the container is closed. Many known containers are relatively durable molded structures intended for repeated use and include replaceable covers.

The reuse of contact lens storage containers over extended periods of time raises the possibility of bacterial contamination of the lens case. In fact, up to 83% of lens cases may be contaminated by pathogenic organisms. This is understood to take place through the formation of biofilms upon the surfaces of the lens case. A biofilm is a type of fouling that occurs when microorganisms attach to surfaces and secrete a hydrated polymeric matrix that surrounds them. Microorganisms existing in a biofilm, termed sessile, grow in a protected environment that insulates them from attack from antimicrobial agents contained in most lens disinfection systems. Biofilms are understood to be a frequently occurring reservoir for infectious agents. The biology of biofilms is described in more detail in "Bacterial biofilms: a common cause of persistent infection," *Costerson, et al*., Science 284: 1318-1322 (1999).

In fact, the contact lens case has been implicated as a factor in microbial keratitis due to the fact that most contact lens disinfection systems are designed to deal with contamination on the contact lens, and not necessarily with contamination existing in the biofilm on the contact lens case. Even as potent of an antimicrobial agent as hydrogen peroxide may be ineffective against an established biofilm. One mechanism of biofilm resistance to antimicrobial agents is the failure of an agent to penetrate the full depth of the biofilm. Polymeric substances like those that make up the matrix of a biofilm are known to retard the diffusion of antimicrobial agents. Because hydrogen peroxide is deactivated in the outer layers of the biofilm faster than it diffuses, the biofilm presents a formidable penetration barrier.

Thus, a disinfected contact lens placed in a contaminated contact lens case may itself become contaminated prior to being placed in the eye. This at least partially negates the beneficial effects of contact lens disinfecting. Contact lens cases are continually exposed to environmental contaminants, as well as to the contact lenses themselves.

Since the difficulties associated with eliminating bacterial contamination of lens cases are well-recognized, a number of technologies have developed to treat lens case surfaces or materials to prevent or impair bacterial contamination. A number of technologies have been proposed that treat surfaces with organic or inorganic materials to interfere with bacterial growth.

US-A-5 515 117 to Anger et al. relates to contact lens cases made from materials which comprise polymeric materials and effective antimicrobial components, said components are covalently bonded to the polymeric materials and substantially non-leachable.

WO 00/38552 to *Barry, et al*. discloses an antimicrobial contact lens case that is comprised of an antimicrobial polymeric resin and has an antimicrobial surface. The contact lens case is made from a polymeric material containing zeolite which has ion-exchanged thereon silver and ammonium ions. The case of the invention does not leach ions from the polymeric material into the ophthalmic solution in a substantially antimicrobial amount.

US-A-.5,320,843 to *Raheja et al*. provides a method for improving the antibacterial efficacy of an ophthalmic solution used in the care of contact lenses. The method uses an article molded from a plastic resin including an aluminosilicate carrier, such as zeolite, retaining antibacterial metal ions. Ophthalmic solution is placed in contact with the article. To render the solution antimicrobial, the zeolite releases relatively large amounts of metal ions into the ophthalmic solution in contact with the case.

US-A-. 5,340,583 to *Dziabo, et al*. teaches a contact lens case containing a non-leachable antimicrobial component that is either deposited on the surface of a pre-formed case or is copolymerized into the matrix of the case when it is formed. In either event, the antimicrobial components do not migrate within the lens case material or between the lens case and the liquid medium it contains. Thus, abrasion of the surface of the lens case will result in an area lacking in antimicrobial protection. Furthermore, the use of antimicrobial halogenated hydrocarbons is not disclosed.

Although several antimicrobial agents exist, the majority are not appropriate for contact with the delicate tissues of the eye on a repeated basis. Furthermore, topical treatments of the lens cases are not durable and are removed through the abrasive process of inserting and removing the lens from the case. What is needed is an antimicrobial agent that can be incorporated into the lens case at the time of manufacture, which is free from toxic effect and is durable over the lifespan of the case.

### SUMMARY OF THE INVENTION

The present invention provides a novel antimicrobial contact lens container, which is adapted to hold one or more contact lenses in an ophthalmic solution. The contact lens container is made from a polymeric material containing an embedded organic antimicrobial agent that does not chemically react with the polymeric material of the lens case.

Accordingly, the present invention, in one aspect, relates to a contact lens case having antimicrobial characteristics comprising a polymeric body defining at least one cavity for holding a contact lens, said polymeric body comprising:
(a) a polymeric material and
(b) an organic antimicrobial agent substantially uniformly distributed throughout said body, wherein said antimicrobial agent does not chemically react with said polymeric material and said antimicrobial agent is more polar than said polymeric material.

In another embodiment, the present invention provides a contact lens case having antimicrobial characteristics where the lens case is constructed of a polymeric material free of antimicrobial monomers.

In yet another embodiment of the present invention, a lens case is provided containing a substantially water-insoluble organic antimicrobial agent substantially uniformly distributed throughout the lens case.

In the preferred embodiment, the invention provides contact lens case having antimicrobial characteristics comprising a leaching antimicrobial agent incorporated into amorphous zones of the polymeric material of the lens cases. The antimicrobial agent exhibits controlled migration through the amorphous zones of the polymeric material. Furthermore, the antimicrobial agent will migrate to the surface of the lens case when an imbalance of vapor pressure of the antimicrobial agent demands equalization (e.g., when the surface is abraded). In this manner, the lens case provides a consistent supply of antimicrobial agent at the surface of the lens case.

The present invention also provides a method for storing a contact lens within the lens case of the present invention. The lens is placed within the cavity of the inventive lens case with an isotonic aqueous solution and then closed to outside contamination.

### BRIEF DESCRIPTION OF THE DRAWINGS

Figure 1 is a top perspective view of one embodiment of the contact lens case of the invention.
Figure 2 is a side view, partially in cross section, of the lens case shown in Figure 1 showing the antimicrobial agent dispersed throughout the polymeric material comprising the contact lens case.
Figure 3 is a top perspective view of one embodiment of the contact lens case of the invention.
Figure 4 is a cross-sectional view of the lens case shown in Figure 3 showing the antimicrobial agent dispersed throughout the polymeric material comprising the contact lens case.

### DETAILED DESCRIPTION OF THE PREFERRED EMBODIMENTS

An antimicrobial additive is incorporated into the resin in concentrate form into the amorphous zones of the molecular structure of the polymer from which lens cases are injection molded, thereby incorporating the antimicrobial agent into the lens case. The high levels of antimicrobial additive in the body of the lens case, incorporated in the manner above, results in substantive controlled migration throughout the body of the lens case and to the surface of the lens case, until a point of equilibrium is reached. If the surface of the lens case is abraded during use and this equilibrium is disrupted, additional migration to the surface is stimulated, until equilibrium is again reached.

Incorporating the antimicrobial agent into the polymer during manufacture of the polymer can be difficult because of the high temperatures and varying physical parameters involved. Organic antimicrobial agents typically have a vaporization point less than the temperatures involved during manufacture of the polymer. For example, 5-chloro-2-(2,4-dichlorophenoxy)phenol has a range of liquid phase from about 135 °F to about 165 °F (about 57 to about 74 °C) and a vaporization point of about 400 °F (about 204 °C), whereas the temperatures associated with forming plastic are typically above 400 °F In that respect, if antimicrobial agent is introduced into the polymer during manufacture, the agent typically vaporizes and does not become incorporated into the polymer. Alternatively, the antimicrobial agent may cross-link with the polymer. Cross-linking of the antimicrobial agent with the polymer is undesirable because the physical properties of the polymer can be degraded. Furthermore, cross-linking prevents the migration of antimicrobial agent through the polymer of the lens case and eventually onto the surface of the lens case.

The preferred method of associating the antimicrobial agent with the lens case is to incorporate the agent into a synthetic polymeric master batch containing approximately 10% - 20% active ingredient prior to forming the lens case. In that respect, the antimicrobial agent in concentrate pellet form is added as a component to the mixture comprising the synthetic polymeric material in a let-down ratio which results in a final concentration of active ingredient of from about 1 percent to about 50 percent by weight. However, the exact amount will depend on the antimicrobial agent and the polymer matrix. The preferred master batch contains LDPE combined with 10% triclosan, commercially available as CIBA® IRGAGUARD® B 1110, from Ciba Specialty Chemicals.

By combining pellets from the master batch production with other polymer pellets, the resulting polymer in the lens case that is formed has a known concentration of antimicrobial agent. While the concentration of the antimicrobial agent can be from 0.001 to 10% by weight, a range of from 0.1% to 0.5% of antimicrobial agent in the resulting polymer is preferred. The most preferable range of antimicrobial agent incorporated into the polymer is from 0.15% to 0.25%. The resulting synthetic polymeric mixture is injection molded or formed by any other molding process (e.g., compression or extrusion) to provide the lens case.

Because of the encapsulation of the antimicrobial agent, the antimicrobial agent survives heating process and is incorporated into the amorphous zones of the polymer. The characteristics of the antimicrobial agent allow the agent to migrate through the polymer to the surface of the lens case from the amorphous zones until equilibrium of the agent's internal vapor pressure is reached. If the antimicrobial agent on the surface of the lens case is removed or the lens case is scratched by friction, or other means more antimicrobial agent will move to the surface until the agent's internal vapor pressure is once again at equilibrium. Normally the antimicrobial agent melts at approximately 150 °F, and loses its biocidal properties when heated above 400 °F However, in the present invention, by encapsulating the antimicrobial agent prior to combining or incorporating the agent into the lens case, the antimicrobial agent loses none of its biocidal properties in the formed lens case.

As noted hereinabove, when the antimicrobial agents are added to the polymer while the polymer is in its molten or liquid stage, the agent lodges in an amorphous, or clear zone within the lens case as it hardens. At this point, due to a process known as "solid state migration," the antimicrobial agent migrates to the surface of the lens case until equilibrium is reached in the internal vapor pressure of the polymer. When equilibrium is reached, the migration ceases and the antimicrobial agent is available to control organisms that contact it, or are in its zone of inhibition. In this manner, the surface of the lens case becomes saturated with the antimicrobial agent.

If the surface of the lens case is not abraded, then no additional migration occurs, providing long-term inhibition capability. If abrasion occurs, the process repeats itself, and the surface becomes re-saturated with the antimicrobial agent. Effective inhibition of organisms continues until all of the antimicrobial agent is exhausted. In the case of a lens case, long term durability is expected within the matrix, and on surfaces subject to abrasion.

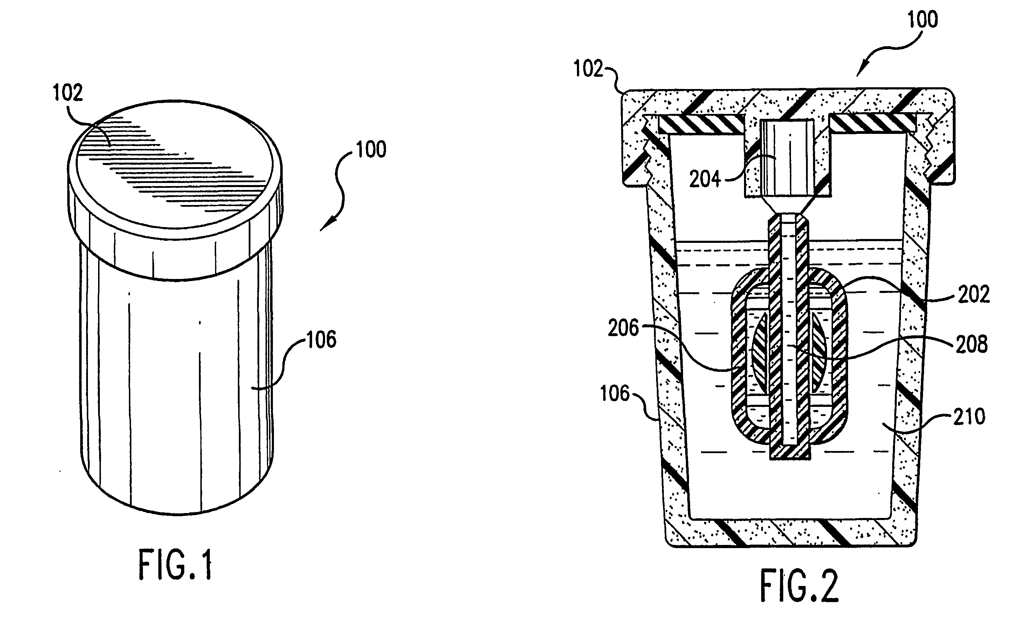

Lens cases according to the invention may be a wide variety of shapes and design. Figures 1 - 4 illustrate only two of a multitude of possible designs. Referring to the figures, Figures 1 and 2 depict one type of contact lens case, shown generally as 100, includes a lens container 106, a cover 102, and a lens basket 208. The top of the lens container may also include a threaded outer surface for engaging a threaded surface inside of the cover 102. The sealable cover 102 may have a basket 206 for holding one or more contact lenses. The basket 206 is attached to the sealable cover 102 by a stem 204 extending from an inner surface of the cap.

A left basket cover 206 and a right basket cover 202 are both hingedly secured to basket body 208 and are structured to be "snapped" closed around left lens mount and right lens mount, respectively, as desired, to form a left lens compartment and a right lens compartment, respectively. The basket covers 206 and 202 may be made separately from the other components of the lens basket 208, or can be molded simultaneously therewith in one piece. Each of the basket covers 206 and 202 includes a series of through holes that allow liquid to flow freely through. However, these through holes are sized so that the contact lenses in lens compartments cannot be removed when the lens covers 206 and 202 are closed. Left lens cover 206 may be marked with "L" to indicate that it is to be used with the left contact lens. Similarly, the right lens cover 202 may be marked with an "R" to indicate that it is to be used with a right contact lens.

Lens container apparatus 100 may be used as follows: with cover 102 removed from lens container 106, the contact lenses, after having been used in the eye are placed on the appropriate left and right lens mounts respectively, and the left and right lens basket covers 206 and 202 are snapped closed. A quantity, e.g., about 10 ml, of a conventional preserved aqueous saline or disinfecting solution is placed in the interior space of lens container 210. Lens case cover 102 is then snapped or screwed in place to enclose the interior space of lens container 210 and to prevent contamination from the environment in the form of dust or microbial organisms. In this manner, the contact lenses held in lens basket 208 are disinfected or maintained in the disinfected condition, even though they may be stored for a prolonged period of time.

Those of ordinary skill in the art will recognize that the solution placed with the lens cavity 110 can be any aqueous solution compatible with contact lenses. The solution is preferably a preserved isotonic solution. Examples include a preserved saline solution, such as SoftWear Saline; a disinfecting multi-purpose solution such as SOLO-Care®; and a hydrogen peroxide solution such as AOSEPT®, all commercially available from CIBA Vision Corp., of Duluth, Georgia. If a hydrogen peroxide solution is used, the lens container 100 may also contain a metal-catalyst coated disc (not shown) at the base of the lens basket stem 208 or elsewhere within the lens cavity 210 to neutralize the hydrogen peroxide. Such discs are well known in the art and are available commercially as AODisc®, from CIBA Vision. Although it is preferred that lens case cover 102 form a solid barrier to outside contamination, in embodiments having a catalytic disc for decomposition of hydrogen peroxide, it is important that the lens cover 102 provide some form of venting mechanism for the gases formed from peroxide decomposition to escape from the lens cavity 210. Such venting mechanisms are described in US Patent Nos. 4,750,610 and 4,637,919.

When it is desired to use the contact lenses being held in lens basket 208, they may be removed from lens basket 208 after removing cover 102, by simply unsnapping the basket covers 206 and 202, and removing the lenses. Providing that the solution used in lens cavity 210 is properly labeled for such use, the removed lenses can be placed directly in the eye for safe and comfortable wear.

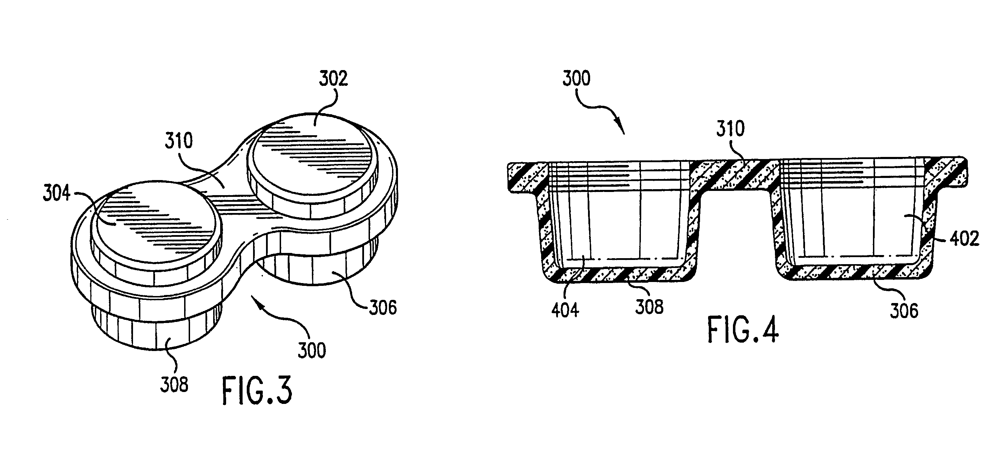

Figures 3 and 4 show another embodiment of the present invention shown generally at 300. The lens case has a left lens holder 308 and right lens holder 306, defining a left lens cavity 404 and a right lens cavity 402 respectively. The lens holders 308 and 306 are joined together 310 to form an integral unit. Each lens cavity has a cover, 304 and 302 respectively. The top of each lens holder 308 and 306 may also include a threaded outer surface for engaging a threaded surface inside of the covers 304 and 302. Left lens cover 304 may be marked with "L" to indicate that it is to be used with the left contact lens. Similarly, the right lens cover 302 may be marked with an "R" to indicate that it is to be used with a right contact lens.

Lens container apparatus 300 may be used as follows: with covers 304 and 302 removed from lens container 300, the contact lenses, after having been used in the eye are placed on the appropriate left and right lens cavities 404 and 402, respectively. A quantity, e.g., about 5 ml, of a conventional preserved aqueous saline or disinfecting solution is placed in each lens cavity 404 and 402. Each cover 304 and 302 is placed upon the appropriate lens cavity and snapped or screwed into place, thus enclosing the cavities 402 and 404 from environmental contamination. In this manner, the contact lenses held in the lens cavities are maintained in the disinfected condition, even though they may be stored for a prolonged period of time.

When it is desired to use the contact lenses being held in lens container 300, they may be removed from their respective cavities 404 and 402 by simply unsnapping or unscrewing the covers 304 and 302, and removing the lenses with a finger. The removed lenses can be placed directly in the eye for safe and comfortable wear.

The plastic resin is preferably a thermoplastic or thermosetting resin suitable for molding, such as injection molding or blow molding. Additionally, the resin is selected such that it is compatible with the antimicrobial compound. One suitable class of resins is thermoplastic polyolefin resins, including high and low density polyethylene and polypropylene. Other suitable resins include polycarbonates, polyvinyl chlorides, polystyrene, such as acrylonitrile butadiene styrene, as well as other plastic resins known in the art. The lens case, basket, and cover may be of the same material, or of different materials.

The crystallinity of the polymer matrix is an important consideration in the design of the antimicrobial lens case of the present invention. The crystallinity of a polymer relates to the orderliness of the three-dimensional arrangement of the polymer within the matrix. Because the antimicrobial agent migrates primarily through the amorphous (or unstructured) zones of the polymer matrix, the antimicrobial agent will more readily migrate to the surface of the lens case if the case is constructed of a polymer of lower crystallinity. However, some degree of crystallinity will be desired to provide for a more controlled migration. The currently preferred polymer is a semi-crystalline polyolefin (having both crystalline and amorphous zones), with semi-crystalline polypropylene being the most preferred.

Ordinarily the parts of the lens cases are formed by injection molding through a conventional plastic injection molding machine. A hopper with starting pellets is conventionally utilized to feed materials to an extruder and then to an injection molding die. Pellets of plastic and of solid anti-microbial active compound in powder form, master-batch, pre-compounded, or any other form are fed to the hopper. Where the active is liquid at room temperature, the anti-microbial may be introduced by liquid injection. Processing temperatures within the extruder portion of the molding machine are maintained in a range of 50 °C to 220 °C, preferably in the range 65 °C to 200 °C.

A wide variety of anti-microbial active compounds, or mixtures thereof, may be employed. These actives may generally be classified as halogenated hydrocarbons, phenolic compounds, benzoic esters, and quaternary ammonium salts.

### I. Halogenated Hydrocarbons

Halogenated hydrocarbons include halogenated derivatives of diphenyl ethers, salicylanilides, carbanilides, mono- and poly-alkyl and aralkyl phenols, bisphenols, anilides of thiophene carboxylic acids and chlorhexidines.

### A. Halogenated Diphenyl Ethers

Halogenated diphenyl ethers will normally contain bromine and/or chlorine, with chlorine being the preferred halogen. They will preferably be substituted with 1 to 3 hydroxyls and 1 to 4 halogens. More preferably they will be substituted with 1 or 2 hydroxyls and 2 or 3 halogens, preferably with four substituents, two on each ring. Among the more preferred of such compounds are 2, 2'-dihydroxy-5,5'-dibromo-diphenyl ether and 5-chloro-2-(2,4-dichlorophenoxy)phenol (triclosan), with the latter compound being most preferred.

### B. Halogenated Salicylanilides

Among the halogenated salicylanilides there may be mentioned the following derivatives: 5-bromo-salicylanilide; 4',5-dibromo-salicylanilide; 3,4',5-tribromo-salicylanilide; 6-chloro-salicylanilide; 4'5-dichloro-salicylanilide; 3,4'5-trichloro-salicylanilide; 4',5-diiodo-salicylanilide; 3,4',5-triiodo-salicylanilide; 5-chloro-3'-trifluoromethyl-salicylanilide; 5-chloro-2'-trifluoromethyl-salicylanilide; 3, 5-dibromo-3'-trifluoromethyl-salicylanilide; 3-chloro-4-bromo-4'-trifluoromethyl-salicylanilide; 2',5-dichloro-3-phenyl-salicylanilide; 3',5-dichloro-4'-methyl-3-phenylsalicylanilide; 3',5-dichloro-4'-phenyl-3-phenyl-salicylanilide; 3,3',5-tri-chloro-6'-(p-chlorophenoxy)-salicylanilide; 3',5-dichloro-5'-(p-bromophenoxy)-salicylanilide; 3,5-dichloro-6'-phenoxy-salicylanilide; 3,5-dichloro-6'-(o-chloro-phenoxy)-salicylanilide; 5-chloro-6'-(o-chlorophenoxy)-salicylanilide; 5-chloro-6'-beta-naphthyloxy-salicylanilide; 5-chloro-6'-alpha-naphthyloxy-salicylanilide; 3,3', 4-trichloro-5,6'-beta-naphthyloxy-salicylalide.

### C. Halogenated Carbanilides

Halogenated carbanilides are represented by the 3,4,4'-trichloro- carbanilide (triclocarban) and the 3,3',4-trichloro derivatives and by 3- trifluoromethyl-4,4'-dichlorocarbanilide.

### D. Halogenated Mono- And Poly-Alkyl And Aralkyl Phenols

Examples of mono- and poly-alkyl and aralkyl phenols include methyl-p-chlorophenol; ethyl-p-chlorophenol; n-propyl-p-chlorophenol; n-butyl-p-chlorophenol; n-amyl-p-chlorophenol; sec-amyl-p-chlorophenol; n-hexyl-p-chlorophenol; cyclohexyl-p-chlorophenol; n-heptyl-p-chlorophenol; n-octyl-p-chlorophenol; o-chlorophenol; methyl-o-chlorophenol; ethyl-o-chlorophenol; n-propyl-o-chlorophenol; n-butyl-o-chlorophenol; n-amyl-o-chlorophenol; tert-amyl-o-chlorophenol; n-hexyl-o-chlorophenol; n-heptyl-o-chlorophenol; p-chlorophenol; o-benzyl-p-chlorophenol; o-benzyl-m-methyl-p-chlorophenol; o-benzyl-m, m-dimethyl-p-chlorophenol; o-phenylethyl-p-chlorophenol; o-phenylethyl-m-methyl-p-chlorophenol; 3-methyl-p-chlorophenol; 3,5-dimethyl-p-chlorophenol; 6-ethyl-3-methyl-p-chlorophenol; 6-n-propyl-3-methyl-p-chlorophenol; 6-iso-propyl-3-methyl-p-chlorophenol; 2-ethyl-3,5-dimethyl-p-chlorophenol; 6-sec butyl-3-methyl-p-chlorophenol; 6-diethylmethyl-3-methyl-p-chlorophenol; 6-iso-propyl-2-ethyl-3-methyl- p-chlorophenol; 2-sec amyl-3,5-dimethyl-p-chlorophenol; 2-diethylmethyl-3,5-dimethyl-p-chlorophenol; 6-sec octyl-3-methyl-p-chlorophenol; p-bromophenol; methyl-p-brdmophenol; ethyl-p-bromophenol; n-propyl-p-bromophenol; n-butyl-p-bromophenol; n-amyl-p-bromophenol; sec-amyl-p-bromophenol; n-hexyl-p-bromophenol; cyctohexyl-p-bromophenol; o-bromophenol; tert-amyl-o-bromophenol; n-hexyl-o-bromophenol; n-propyl-m,m-dimethyl-o-bromophenol; 2-phenyl phenol; 4-chloro-2-methyl phenol; 4-chloro-3-methyl phenol; 4-chloro-3,5-dimethyl phenol; 2,4-dichloro-3,5-dimethylphenol; 3,4,5,6-terabromo-2-methylphenol; 5-methyl-2-pentylphenol; 4-isopropyl-3-methylphenol; 5-chloro-2-hydroxydiphenylemthane.

### E. Halogenated bis-Phenols

The bis-phenols are represented by the following: 2,2'-methylenebis(4-chlorophenol); 2,2'-methylenebis(4,5-dichlorophenol); 2,2'-methylenebis(3,4,6-tri-chlorophenol); 2,2'-thiobis(4,6-dichlorophenol); 2,2'-dikeiobis(4-bromophenol); 2, 2'-methylenebis(4-chloro-6-isopropylphenol); 2,2'-isopropylidenebis(6-sec-butyl-4-chlorophenol).

### II. Phenolic Compounds

Suitable phenolic compounds include phenol, resorcinol and catechol and their derivatives.

### A. Phenol Derivatives

Suitable phenolic derivatives include 2-methyl-phenol; 3-methyl-phenol; 4-methyl-phenol; 4-ethyl-phenol; 2,4-dimethyl-phenol; 2,5-dimethyl-phenol; 3,4-dimethyl-phenol; 2,6-dimethyl-phenol; 4-n-propyl-phenol; 4-n-butyl-phenol; 4-n-amyl-phenol; 4-tert-amyl-phenol; 4-n-hexyl-phenol; 4-n-heptyl-phenol; 2-methoxy-4-(2-propenyl)-phenol (eugenol); p-2-isopropyl-5-methyl-phenol (thymol).

### B. Resorcinol Derivatives

Examples of suitable resorcinol derivatives include methyl-resorcinol; ethyl-resorcinol; n-propyl-resorcinol; n-butyl-resorcinol; n-amyl-resorcinol; n-hexyl-resorcinol; n-heptyl-resorcinol; n-octyl-resorcinol; n-nonyl-resorcinol; phenyl-resorcinol; benzyl-resorcinol; phenylethyl-resorcinol; phenylpropyl-resorcinol; p-chlorobenzyl-resorcinol; 5-chloro-2,4-dihydroxy-di-phenyl methane; 4'-chloro-2,4-dihydroxydiphenyl methane; 5-bromo-2,4-dihydroxydiphenyl methane; 4'-bromo -2,4-dihydroxydiphenyl methane.

### III. Benzoic Esters

Examples of suitable benzoic esters include methyl-p-hydroxybenzoic ester; ethyl-p-hydroxybenzoic ester; propyl-p-hydroxybenzoic ester; and butyl-p-hydroxybenzoic ester.

### IV. Quaternary Ammonium Compounds

Quaternary ammonium compounds include alkyl ammonium, pyridinum, and isoquinolinium salts. Examples of quaternary ammonium compounds are: cetyl pyridinium chloride; diisobutylphenoxyethoxyethyldimethylbenzyl ammonium chloride; N-methyl-N-(2-hydroxyethyl)-N-(2-hydroxydodecyl)-N-benzyl ammonium chloride; cetyl trimethylammonium bromide; stearyl trimethylammonium bromide; oleyl dimethylethylammonium bromide; lauryldimethylchlorethoxyethylammonium chloride; lauryldimethylbenzyl-ammonium chloride; alkyl (C₈-C₁₈) dimethyl(3,4-dichlorobenzyl)-ammonium chloride; lauryl pyridinium bromide; lauryl iso-quinolinium bromide; N(lauroyloxyethylaminoformylmethyl) pyridinium chloride.

The antibacterial agent is preferably a non-cationic compound, which is water insoluble or essentially water insoluble (having a solubility in water at 25 °C of less than 10 g/l, and preferably less than 1 or 0.1 g/l). The low solubility of the antibacterial agent prevents substantial migration of the compound into the aqueous matrix during use of the lens case.

From the viewpoint of safety and effectiveness the preferred antibacterial agents are 4',5-dibromosalicylanilide; 3,4',5-tribromosalicylanilide; 3,4',5-trichlorosalicylanilide; 3,4,4'-trichlorocarbanilide; 3-trifluoromethyl4,4'-dichloro-carbanilide; 2,2'-methylenebis(3,4,6-trichlorophenol); 5-chloro-2-(2,4-dichloro-phenoxy) phenol; Tyrothricin; N-methyl-N-(2-hydroxyethyl-N-(2-hydroxydodecyl)-N-benzylammonium chloride; cetyl pyridinium chloride.

Especially preferred are 2,3'5-tribromosalicylanilide; chlorohexidine digluconate; chlorohexidine diaceate; 4',5-dibromosalicylanilide; 3,4,4'-trichlorocarbanilide; 5-chloro-2-(2,4-dichlorophenoxy)phenol; cetyl pyridinium chloride.

Most preferred among the halogenated hydrocarbons is 5-chloro-2-(2,4- dichloro-phenoxy)phenol (TRICLOSAN) and among the quaternary ammonium salts is cetyl pyridinium chloride. Indeed, combinations of both of these active substances allow for a broad range of anti-microbial activity in the lens cases of the present invention. The most preferred compound is 5-chloro-2-(2,4- dichlorophenoxy)phenol.

However, when selecting the antimicrobial agent, the polymeric material of the lens case body should be considered. Specifically, the antimicrobial agent should be more polar than the lens case polymer. The more polar the antimicrobial agent, the more effective the migration to the surface of the lens case. In this respect, it is preferable for the antimicrobial agent to have a greater dipole moment than polymer matrix of the lens case body. If the polarity of the antimicrobial is the same as the polymer matrix, the above-mentioned migration of the antimicrobial agent to the surface will not occur.

The antimicrobial agent included in the contact lens case should preferably be ophthalmically acceptable. That is, such antimicrobial agent should be selected so as to have, or to cause, no significant adverse effect on the wearer, in particular on the ocular health of the wearer of a contact lens stored in a lens case of the present invention.

However, due to the minimal amounts of the agent that the user may come into contact with during use of the present invention, the toxicity of the antimicrobial agent is not expected to be of major concern.

## Claims

1. A contact lens case having antimicrobial characteristics comprising a polymeric body defining at least one cavity for holding a contact lens
**characterized in that** said polymeric body comprising:
(a) a polymeric material and
(b) an organic antimicrobial agent substantially uniformly distributed throughout said body,
wherein said antimicrobial agent does not chemically react with said polymeric material and said antimicrobial agent is more polar than said polymeric material.

2. A contact lens case according to Claim 1, wherein said polymeric material is a semi-crystalline polyolefin.

3. A contact lens case according to Claim 1, wherein said antimicrobial agent is selected from the group consisting of halogenated hydrocarbons, phenolic compounds, benzoic esters, and quaternary ammonium salts.

4. A contact lens case according to Claim 3, wherein said antimicrobial agent is a halogenated hydrocarbon selected from the group consisting of halogenated diphenyl ethers, halogenated salicylanilides, halogenated carbanilides, halogenated mono- and poly-alkyl and aralkyl phenols, and halogenated bisphenols.

5. A contact lens case according to Claim 4, wherein said antimicrobial agent is a halogenated diphenyl ether substituted with 1 to 3 hydroxyls and 1 to 4 halogens selected from the group consisting of bromine and chlorine.

6. A contact lens case according to Claim 5, wherein said antimicrobial agent is triclosan.

7. A contact lens case according to Claim 1, wherein said antimicrobial agent is selected from the group consisting of 4',5-dibromosalicylanilide; 3,4',5-tribromosalicylanilide; 3,4',5-trichlorosalicylanilide; 3,4,4'-trichlorocarbanilide; 3-trifluoromethyl-4,4'-dichloro-carbanilide; 2,2'-methylenebis(3,4,6-trichlorophenol); 5-chloro-2-(2,4-dichloro-phenoxy) phenol; Tyrothricin; N-methyl-N-(2-hydroxyethyl-N-(2-hydroxydodecyl)-N-benzylammonium chloride; cetyl pyridinium chloride.

8. A contact lens case according to Claim 1, wherein said antimicrobial agent has a solubility in water at 25 °C of less than 10 g/l.

9. A contact lens case according to claim 1, wherein said polymeric body is composed of a polymeric material substantially free of antimicrobial monomers.

10. A contact lens case according to Claim 9, wherein said polymeric material is a semi-crystalline polyolefin.

11. A contact lens case according to Claim 9 or 10, wherein said antimicrobial agent is selected from the group consisting of halogenated hydrocarbons, phenolic compounds, benzoic esters, and quaternary ammonium salts.

12. A contact lens case according to Claim 11, wherein said antimicrobial agent is a halogenated hydrocarbon selected from the group consisting of halogenated diphenyl ethers, halogenated salicylanilides, halogenated carbanilides, halogenated mono- and poly-alkyl and aralkyl phenols, and halogenated bisphenols.

13. A contact lens case according to Claim 12, wherein said halogenated diphenyl ether is a diphenyl ether substituted with 1 to 3 hydroxyls and 1 to 4 halogens selected from the group consisting of bromine and chlorine.

14. A contact lens case according to Claim 13, wherein said antimicrobial agent is triclosan.

15. A contact lens case according to Claim 9, wherein said antimicrobial agent is selected from the group consisting of 4',5-dibromosalicylanilide; 3,4',5-tribromosalicylanilide; 3,4',5-trichlorosalicylanilide; 3,4,4'-trichlorocarbanilide; 3-trifluoromethyl-4,4'-dichloro-carbanilide; 2,2'-methylenebis(3,4,6-trichlorophenol); 5-chloro-2-(2,4-dichloro-phenoxy) phenol; Tyrothricin; N-methyl-N-(2-hydroxyethyl-N-(2-hydroxydodecyl)-N-benzylammonium chloride; cetyl pyridinium chloride.

16. A contact lens case according to Claim 9, wherein said antimicrobial agent has a solubility in water at 25 °C of less than 10 g/l.

17. A contact lens case according to claim 1, wherein said organic antimicrobial agent is substantially water-insoluble.

18. A contact lens case according to Claim 17, wherein said antimicrobial agent has a solubility in water at 25 °C of less than 1 g/l.

19. A contact lens case according to Claim 17, wherein said antimicrobial agent is selected from the group consisting of halogenated hydrocarbons, phenolic compounds, benzoic esters, and quaternary ammonium salts.

20. A contact lens case according to Claim 19, wherein said antimicrobial agent is a halogenated hydrocarbon selected from the group consisting of halogenated diphenyl ethers, halogenated salicylanilides, halogenated carbanilides, halogenated mono- and poly-alkyl and aralkyl phenols, and halogenated bisphenols.

21. A contact lens case according to Claim 20, wherein said antimicrobial agent is a halogenated diphenyl ether substituted with 1 to 3 hydroxyls and 1 to 4 halogens selected from the group consisting of bromine and chlorine.

22. A contact lens case according to Claim 21, wherein said antimicrobial agent is triclosan.

23. A contact lens case according to Claim 17, wherein said antimicrobial agent is selected from the group consisting of 4',5-dibromosalicylanilide; 3,4',5-tribromosalicylanilide; 3,4',5-trichlorosalicylanilide; 3,4,4'-trichlorocarbanilide; 3-trifluoromethyl-4,4'-dichloro-carbanilide; 2,2'-methylenebis(3,4,6-trichlorophenol); 5-chloro-2-(2,4-dichloro-phenoxy) phenol; Tyrothricin; N-methyl-N-(2-hydroxyethyl-N-(2-hydroxydodecyl)-N-benzylammonium chloride; cetyl pyridinium chloride.

24. A contact lens case according to claim 1, wherein said polymeric body is composed of a polymeric material having a plurality of amorphous zones; wherein said antimicrobial agent is incorporated into said amorphous zones of said polymeric material of said body, and wherein said antimicrobial agent exhibits controlled migration through said amorphous zones of said polymeric material of said body.

25. A contact lens case according to Claim 24, wherein said antimicrobial agent exhibits controlled migration through said amorphous zones of said polymeric material of said body to the surface thereof when an imbalance of vapor pressure of said antimicrobial agent demands equalization.

26. A contact lens case according to Claim 24, wherein said antimicrobial agent is selected from the group consisting of halogenated hydrocarbons, phenolic compounds, benzoic esters, and quaternary ammonium salts.

27. A contact lens case according to Claim 26, wherein said antimicrobial agent is a halogenated hydrocarbon selected from the group consisting of halogenated diphenyl ethers, halogenated salicylanilides, halogenated carbanilides, halogenated mono- and poly-alkyl and aralkyl phenols, and halogenated bisphenols.

28. A contact lens case according to Claim 27, wherein said antimicrobial agent is a halogenated diphenyl ether substituted with 1 to 3 hydroxyls and 1 to 4 halogens selected from the group consisting of bromine and chlorine.

29. A contact lens case according to Claim 28, wherein said antimicrobial agent is triclosan.

30. A contact lens case according to Claim 24, wherein said antimicrobial agent is selected from the group consisting of 4',5-dibromosalicylanilide; 3,4',5-tribromosalicylanilide; 3,4',5-trichlorosalicylanilide; 3,4,4'-trichlorocarbanilide; 3-trifluoromethyl-4,4'-dichloro-carbanilide; 2,2'-methylenebis(3,4,6-trichlorophenol); 5-chloro-2-(2,4-dichloro-phenoxy) phenol; Tyrothricin; N-methyl-N-(2-hydroxyethyl-N-(2-hydroxydodecyl)-N-benzylammonium chloride; cetyl pyridinium chloride.

31. A contact lens case according to Claim 24, wherein said antimicrobial agent has a solubility in water at 25 °C of less than 10 g/l.

32. A contact lens case according to claim 1,
wherein said polymeric material is a semi-crystalline polyolefin having a plurality of amorphous zones;
wherein said antimicrobial agent is a halogenated diphenyl ether, and
wherein said diphenyl ether exhibits controlled migration through said amorphous zones of said body to the surface thereof when an imbalance of vapor pressure of said diphenyl ether demands equalization.

33. A contact lens case according to any one of the claims 1 to 32, wherein said antimicrobial agent is present in a concentration of from 0.001 to 10 % by weight, preferably of from 0.1 to 0.5 %, in particular of from 0.15 t0 0.25 %.

34. A method for storing a contact lens comprising the steps of
providing a contact lens case having antimicrobial characteristics comprising a polymeric body defining at least one cavity for holding said contact lens
**characterized in that**
a halogenated diphenyl ether is uniformly distributed throughout said body, said polymeric body being composed of a semi-crystalline polyolefin having a plurality of amorphous zones; and wherein said diphenyl ether exhibits controlled migration through said amorphous zones of said body to the surface thereof when an imbalance of vapor pressure of said diphenyl ether demands equalization; and
placing said contact lens and an isotonic aqueous solution into said cavity; and
enclosing said cavity as to prevent environmental contamination.

35. A method for storing a contact lens comprising the steps of
providing a contact lens case as claimed in Claim 1;
placing said contact lens and an isotonic aqueous solution into said cavity; and
enclosing said cavity as to prevent environmental contamination.

36. A method for storing a contact lens comprising the steps of
providing a contact lens case as claimed in Claim 24;
placing said contact lens and an isotonic aqueous solution into said cavity; and enclosing said cavity as to prevent environmental contamination.

## Patentansprüche

1. Kontaktlinsenbehälter mit antimikrobiellen Eigenschaften, umfassend einen Polymerkörper, der mindestens einen Hohlraum zum Halten einer Kontaktlinse definiert, **dadurch gekennzeichnet, dass** der Polymerkörper umfasst:
(a) ein Polymermaterial und
(b) ein organisches antimikrobielles Mittel, das im Wesentlichen gleichförmig durch den Körper verteilt ist, wobei das antimikrobielle Mittel chemisch nicht mit dem Polymermaterial reagiert und das antimikrobielle Mittel polarer als das Polymermaterial ist.

2. Kontaktlinsenbehälter nach Anspruch 1, worin das Polymermaterial ein semikristallines Polyolefin ist.

3. Kontaktlinsenbehälter nach Anspruch 1, worin das antimikrobielle Mittel aus der Gruppe, bestehend aus halogenierten Kohlenwasserstoffen, phenolischen Verbindungen, Benzoesäureestern und quaternären Ammoniumsalzen, ausgewählt ist.

4. Kontaktlinsenbehälter nach Anspruch 3, worin das antimikrobielle Mittel ein halogenierter Kohlenwasserstoff ist, ausgewählt aus der Gruppe, bestehend aus halogenierten Diphenylethern, halogenierten Salicylaniliden, halogenierten Carbaniliden, halogenierten Mono- und Polyalkylund -aralkylphenolen und halogenierten Bisphenolen.

5. Kontaktlinsenbehälter nach Anspruch 4, worin das antimikrobielle Mittel ein halogenierter Diphenylether, substituiert mit 1 bis 3 Hydroxylen und 1 bis 4 Halogenen, ausgewählt aus der Gruppe, bestehend aus Brom und Chlor, darstellt.

6. Kontaktlinsenbehälter nach Anspruch 5, worin das antimikrobielle Mittel Triclosan ist.

7. Kontaktlinsenbehälter nach Anspruch 1, worin das antimikrobielle Mittel aus der Gruppe, bestehend aus 4',5-Dibromsalicylanilid; 3,4',5-Tribromsalicylanilid; 3,4',5-Trichlorsalicylanilid; 3,4,4'-Trichlorcarbanilid; 3-Trifluormethyl-4,4'-dichlor-carbanilid; 2,2'-Methylenbis-(3,4,6-trichlorphenol); 5-Chlor-2-(2,4-dichlor-phenoxy)-phenol; Tyrothricin; N-Methyl-N-(2-hydroxyethyl-N-(2-hydroxydodecyl)-N-benzylammoniumchlorid; Cetylpyridiniumchlorid, ausgewählt ist.

8. Kontaktlinsenbehälter nach Anspruch 1, worin das antimikrobielle Mittel eine Löslichkeit in Wasser bei 25°C von weniger als 10 g/l aufweist.

9. Kontaktlinsenbehälter nach Anspruch 1, worin der Polymerkörper aus einem Polymermaterial zusammengesetzt ist, das im Wesentlichen frei von antimikrobiellen Monomeren ist.

10. Kontaktlinsenbehälter nach Anspruch 9, worin das Polymermaterial ein semikristallines Polyolefin ist.

11. Kontaktlinsenbehälter nach Anspruch 9 oder 10, worin das antimikrobielle Mittel aus der Gruppe, bestehend aus halogenierten Kohlenwasserstoffen, phenolischen Verbindungen, Benzoesäureestern und quaternären Ammoniumsalzen, ausgewählt ist.

12. Kontaktlinsenbehälter nach Anspruch 11, worin das antimikrobielle Mittel einen halogenierten Kohlenwasserstoff, ausgewählt aus der Gruppe, bestehend aus halogenierten Diphenylethern, halogenierten Salicylaniliden, halogenierten Carbaniliden, halogenierten Mono- und Polyalkyl- und -aralkylphenolen und halogenierten Bisphenolen, darstellt.

13. Kontaktlinsenbehälter nach Anspruch 12, worin der halogenierte Diphenylether ein Diphenylether ist, der mit 1 bis 3 Hydroxylen und 1 bis 4 Halogenen, ausgewählt aus der Gruppe, bestehend aus Brom und Chlor, substituiert ist.

14. Kontaktlinsenbehälter nach Anspruch 13, worin das antimikrobielle Mittel Triclosan ist.

15. Kontaktlinsenbehälter nach Anspruch 9, worin das antimikrobielle Mittel aus der Gruppe, bestehend aus 4',5-Dibromsalicylanilid; 3,4',5-Tribromsalicylanilid; 3,4',5-Trichlorsalicylanilid; 3,4,4'-Trichlorcarbanilid; 3-Trifluormethyl-4,4'-dichlor-carbanilid; 2,2'-Methylenbis-(3,4,6-trichlorphenol); 5-Chlor-2-(2,4-dichlor-phenoxy)-phenol; Tyrothricin; N-Methyl-N-(2-hydroxyethyl-N-(2-hydroxydodecyl)-N-benzylammoniumchlorid; Cetylpyridiniumchlorid, ausgewählt ist.

16. Kontaktlinsenbehälter nach Anspruch 9, worin das antimikrobielle Mittel eine Löslichkeit in Wasser bei 25°C von weniger als 10 g/l aufweist.

17. Kontaktlinsenbehälter nach Anspruch 1, worin das antimikrobielle Mittel im Wesentlichen in Wasser unlöslich ist.

18. Kontaktlinsenbehälter nach Anspruch 17, worin das antimikrobielle Mittel eine Löslichkeit in Wasser bei 25°C von weniger als 1 g/l aufweist.

19. Kontaktlinsenbehälter nach Anspruch 17, wobei das antimikrobielle Mittel aus der Gruppe, bestehend aus halogenierten Kohlenwasserstoffen, phenolischen Verbindungen, Benzoesäureestern und quaternären Ammoniumsalzen, ausgewählt ist.

20. Kontaktlinsenbehälter nach Anspruch 19, worin das antimikrobielle Mittel einen halogenierten Kohlenwasserstoff, ausgewählt aus der Gruppe, bestehend aus halogenierten Diphenylethern, halogenierten Salicylaniliden, halogenierten Carbaniliden, halogenierten Mono- und Polyalkyl- und -aralkylphenolen und halogenierten Bisphenolen, darstellt.

21. Kontaktlinsenbehälter nach Anspruch 20, worin das antimikrobielle Mittel ein halogenierter Diphenylether ist, der mit 1 bis 3 Hydroxylen und 1 bis 4 Halogenen, ausgewählt aus der Gruppe, bestehend aus Brom und Chlor, substituiert ist.

22. Kontaktlinsenbehälter nach Anspruch 21, worin das antimikrobielle Mittel Triclosan ist.

23. Kontaktlinsenbehälter nach Anspruch 17, worin das antimikrobielle Mittel aus der Gruppe, bestehend aus 4',5-Dibromsalicylanilid; 3,4',5-Tribromsalicylanilid; 3,4',5-Trichlorsalicylanilid; 3,4,4'-Trichlorcarbanilid; 3-Trifluormethyl-4,4'-dichlor-carbanilid; 2,2'-Methylenbis-(3,4,6-trichlorphenol); 5-Chlor-2-(2,4-dichlor-phenoxy)-phenol; Tyrothricin; N-Methyl-N-(2-hydroxyethyl-N-(2-hydroxydodecyl)-N-benzylammoniumchlorid; Cetylpyridiniumchlorid, ausgewählt ist.

24. Kontaktlinsenbehälter nach Anspruch 1, worin der Polymerkörper zusammengesetzt ist aus einem Polymermaterial mit einer Vielzahl von amorphen Zonen; wobei das antimikrobielle Mittel in die amorphen Zonen des Polymermaterials des Körpers eingearbeitet ist und wobei das antimikrobielle Mittel gesteuerte Migration durch die amorphen Zonen des Polymermaterials des Körpers zeigt.

25. Kontaktlinsenbehälter nach Anspruch 24, worin das antimikrobielle Mittel gesteuerte Migration durch die amorphen Zonen des Polymermaterials des Körpers zu dessen Oberfläche zeigt, wenn ein Ungleichgewicht von Dampfdruck von dem antimikrobiellen Mittel Gleichgewichtseinstellung erfordert.

26. Kontaktlinsenbehälter nach Anspruch 24, worin das antimikrobielle Mittel aus der Gruppe, bestehend aus halogenierten Kohlenwasserstoffen, phenolischen Verbindungen, Benzoesäureestern und quaternären Ammoniumsalzen, ausgewählt ist.

27. Kontaktlinsenbehälter nach Anspruch 26, worin das antimikrobielle Mittel einen halogenierten Kohlenwasserstoff, ausgewählt aus der Gruppe, bestehend aus halogenierten Diphenylethern, halogenierten Salicylaniliden, halogenierten Carbaniliden, halogenierten Mono- und Polyalkyl- und -aralkylphenolen und halogenierten Bisphenolen, darstellt.

28. Kontaktlinsenbehälter nach Anspruch 27, worin das antimikrobielle Mittel ein halogenierter Diphenylether ist, der mit 1 bis 3 Hydroxylen und 1 bis 4 Halogenen, ausgewählt aus der Gruppe, bestehend aus Brom und Chlor, substituiert ist.

29. Kontaktlinsenbehälter nach Anspruch 28, worin das antimikrobielle Mittel Triclosan ist.

30. Kontaktlinsenbehälter nach Anspruch 24, worin das antimikrobielle Mittel aus der Gruppe, bestehend aus 4',5-Dibromsalicylanilid; 3,4',5-Tribromsalicylanilid; 3,4',5-Trichlorsalicylanilid; 3,4,4'-Trichlorcarbanilid; 3-Trifluormethyl-4,4'-dichlor-carbanilid; 2,2'-Methylenbis-(3,4,6-trichlorphenol); 5-Chlor-2-(2,4-dichlor-phenoxy)-phenol; Tyrothricin; N-Methyl-N-(2-hydroxyethyl-N-(2-hydroxydodecyl)-N-benzylammoniumchlorid; Cetylpyridiniumchlorid, ausgewählt ist.

31. Kontaktlinsenbehälter nach Anspruch 24, worin das antimikrobielle Mittel eine Löslichkeit in Wasser bei 25°C von weniger als 10 g/l aufweist.

32. Kontaktlinsenbehälter nach Anspruch 1, worin das polymere Material ein semikristallines Polyolefin mit einer Vielzahl von amorphen Zonen ist,
wobei das antimikrobielle Mittel einen halogenierten Diphenylether darstellt und wobei der Diphenylether gesteuerte Migration durch die amorphen Zonen des Körpers zu dessen Oberfläche zeigt, wenn ein Ungleichgewicht von Dampfdruck des Diphenylethers Gleichgewichtseinstellung erfordert.

33. Kontaktlinsenbehälter nach einem der Ansprüche 1 bis 32, worin das antimikrobielle Mittel in einer Konzentration von 0,001 bis 10 Gewichtsprozent, vorzugsweise 0,1 bis 0,5%, insbesondere 0,15 bis 0,25%, vorliegt.

34. Verfahren zum Lagern einer Kontaktlinse, umfassend die Schritte von
Bereitstellen eines Kontaktlinsenbehälters mit antimikrobiellen Eigenschaften, umfassend einen Polymerkörper, der mindestens einen Hohlraum zum Halten der Kontaktlinse definiert, **dadurch gekennzeichnet, dass** ein halogenierter Diphenylether gleichförmig durch den Körper verteilt ist,
wobei der Polymerkörper aus einem semikristallinen Polyolefin mit einer Vielzahl von amorphen Zonen zusammengesetzt ist, und wobei der Diphenylether gesteuerte Migration durch die amorphen Zonen des Körpers zu dessen Oberfläche zeigt, wenn ein Ungleichgewicht von Dampfdruck des Diphenylethers Gleichgewichtseinstellung erfordert; und
Anordnen der Kontaktlinse und einer isotonischen wässrigen Lösung in dem Hohlraum; und
Einschließen des Hohlraums, um Umweltverunreinigung zu verhindern.

35. Verfahren zum Lagern einer Kontaktlinse, umfassend die Schritte von
Bereitstellen eines Kontaktlinsenbehälters nach Anspruch 1,
Anordnen der Kontaktlinse und einer isotonischen wässrigen Lösung in dem Hohlraum und
Einschließen des Hohlraums, um Umweltverunreinigung zu verhindern.

36. Verfahren zum Lagern einer Kontaktlinse, umfassend die Schritte von
Bereitstellen eines Kontaktlinsenbehälters nach Anspruch 24,
Anordnen der Kontaktlinse und einer isotonischen wässrigen Lösung in dem Hohlraum und
Einschließen des Hohlraums, um Umweltverunreinigung zu verhindern.

## Revendications

1. Etui de lentille de contact ayant des caractéristiques antimicrobiennes, comprenant un corps polymère définissant au moins une cavité pour maintenir une lentille de contact, **caractérisé en ce que** ledit corps polymère comprend :
(a) une matière polymère, et
(b) un agent antimicrobien organique sensiblement uniformément distribué à travers ledit corps, ledit agent antimicrobien ne réagissant pas chimiquement avec ladite matière polymère et ledit agent antimicrobien étant plus polaire que ladite matière polymère.

2. Etui de lentille de contact selon la revendication 1, dans lequel ladite matière polymère est une polyoléfine semi-cristalline.

3. Etui de lentille de contact selon la revendication 1, dans lequel ledit agent antimicrobien est choisi dans le groupe constitué par des hydrocarbures halogénés, des composés phénoliques, des esters benzoïques et des sels d'ammonium quaternaire.

4. Etui de lentille de contact selon la revendication 3, dans lequel ledit agent antimicrobien est un hydrocarbure halogéné choisi dans le groupe constitué par des diphényléthers halogénés, des salicylanilides halogénés, des carbanilides halogénés, des mono- et poly-alkyl- et aralkyl-phénols halogénés, et des bisphénols halogénés.

5. Etui de lentille de contact selon la revendication 4, dans lequel ledit agent antimicrobien est un diphényléther halogéné substitué par 1 à 3 groupes hydroxyle et 1 à 4 halogènes choisis dans le groupe constitué par le brome et le chlore.

6. Etui de lentille de contact selon la revendication 5, dans lequel ledit agent antimicrobien est le triclosan.

7. Etui de lentille de contact selon la revendication 1, dans lequel ledit agent antimicrobien est choisi dans le groupe constitué par le 4',5-dibromosalicylanilide ; le 3,4',5-tribromosalicylanilide ; le 3,4',5-trichloro-salicylanilide ; le 3,4,4'-trichlorocarbanilide ; le 3-trifluorométhyl-4,4'-dichlorocarbanilide ; le 2,2'-méthylènebis(3,4,6-trichlorophénol); le 5-chloro-2-(2,4-dichlorophénoxy)phénol; la Tyrothricine ; le chlorure de N-méthyl-N-(2-hydroxyéthyl-N-(2-hydroxydodécyl)-N-benzylammonium ; le chlorure de cétylpyridinium.

8. Etui de lentille de contact selon la revendication 1, dans lequel ledit agent antimicrobien a une solubilité dans l'eau à 25 °C inférieure à 10 g/litre.

9. Etui de lentille de contact selon la revendication 1, dans lequel ledit corps polymère est composé d'une matière polymère sensiblement dépourvue de monomères antimicrobiens.

10. Etui de lentille de contact selon la revendication 9, dans lequel ladite matière polymère est une polyoléfine semi-cristalline.

11. Etui de lentille de contact selon la revendication 9 ou 10, dans lequel ledit agent antimicrobien est choisi dans le groupe constitué par des hydrocarbures halogénés, des composés phénoliques, des esters benzoïques, et des sels d'ammonium quaternaire.

12. Etui de lentille de contact selon la revendication 11, dans lequel ledit agent antimicrobien est un hydrocarbure halogéné choisi dans le groupe constitué par des diphényléthers halogénés, des salicylanilides halogénés, des carbanilides halogénés, des mono- et poly-alkyl- et aralkyl-phénols, et des bisphénols halogénés.

13. Etui de lentille de contact selon la revendication 12, dans lequel ledit diphényléther halogéné est un diphényléther substitué par 1 à 3 groupes hydroxyles et 1 à 4 halogènes choisis dans le groupe constitué par le brome et le chlore.

14. Etui de lentille de contact selon la revendication 13, dans lequel ledit agent antimicrobien est le triclosan.

15. Etui de lentille de contact selon la revendication 9, dans lequel ledit agent antimicrobien est choisi dans le groupe constitué par le 4',5-dibromosalicylanilide; le 3,4',5-tribromosalicylanilide; le 3,4',5-trichloro-salicylanilide; le 3,4,4'-trichlorocarbanilide ; le 3-trifluorométhyl-4,4'-dichlorocarbanilide; le 2,2'-méthylènebis(3,4,6-trichlorophénol); le 5-chloro-2-(2,4-dichlorophénoxy)-phénol ; la Tyrothricine ; le chlorure de N-méthyl-N-(2-hydroxyéthyl)-N-(2-hydroxydodécyl)-N-benzylammonium ; le chlorure de cétylpyridinium.

16. Etui de lentille de contact selon la revendication 9, dans lequel ledit agent antimicrobien a une solubilité dans l'eau à 25 °C inférieure à 10 g/litre.

17. Etui de lentille de contact selon la revendication 1, dans lequel ledit agent antimicrobien organique est sensiblement insoluble dans l'eau.

18. Etui de lentille de contact selon la revendication 17, dans lequel ledit agent antimicrobien a une solubilité dans l'eau à 25 °C inférieure à 1 g/litre.

19. Etui de lentille de contact selon la revendication 17, dans lequel ledit agent antimicrobien est choisi dans le groupe constitué par des hydrocarbures halogénés, des composés phénoliques, des esters benzoïques et des sels d'ammonium quaternaire.

20. Etui de lentille de contact selon la revendication 19, dans lequel ledit agent antimicrobien est un hydrocarbure halogéné choisi dans le groupe constitué par des diphényléthers halogénés, des salicylanilides halogénés, des carbanilides halogénés, des mono- et poly- alkyl- et aralkyl-phénols halogénés, et des bisphénols halogénés.

21. Etui de lentille de contact selon la revendication 20, dans lequel ledit agent antimicrobien est un diphényléther halogéné substitué par 1 à 3 groupes hydroxyles et 1 à 4 halogènes choisis dans le groupe constitué par le brome et le chlore.

22. Etui de lentille de contact selon la revendication 21, dans lequel ledit agent antimicrobien est le triclosan.

23. Etui de lentille de contact selon la revendication 17, dans lequel ledit agent antimicrobien est choisi dans le groupe constitué par le 4',5-dibromosalicylanilide ; le 3,4',5-tribromosalicylanilide ; le 3,4',5-trichloro-salicylanilide; le 3,4,4'-trichlorocarbanilide ; le 3-trifluorométhyl-4,4'-dichlorocarbanilide; le 2,2'-méthylènebis(3,4,6-trichlorophénol); le 5-chloro-2-(2,4-dichlorophénoxy)-phénol; la Tyrothricine ; le chlorure de N-méthyl-N-(2-hydroxyéthyl)-N-(2-hydroxydodécyl)-N-benzylammonium; le chlorure de cétylpyridinium.

24. Etui de lentille de contact selon la revendication 1, dans lequel ledit corps polymère est composé d'une matière polymère ayant une pluralité de zones amorphes ; dans lequel ledit agent antimicrobien est incorporé dans lesdites zones amorphes de ladite matière polymère dudit corps, et dans lequel ledit agent antimicrobien présente une migration contrôlée à travers lesdites zones amorphes de ladite matière polymère dudit corps.

25. Etui de lentille de contact selon la revendication 24, dans lequel ledit agent antimicrobien présente une migration contrôlée à travers lesdites zones amorphes de ladite matière polymère dudit corps vers la surface de celui-ci lorsqu'un déséquilibre de la pression de vapeur dudit agent antimicrobien requiert une égalisation.

26. Etui de lentille de contact selon la revendication 24, dans lequel ledit agent antimicrobien est choisi dans le groupe constitué par des hydrocarbures halogénés, des composés phénoliques, des esters benzoïques, et des sels d'ammonium quaternaire.

27. Etui de lentille de contact selon la revendication 26, dans lequel ledit agent antimicrobien est un hydrocarbure halogéné choisi dans le groupe constitué par des diphényléthers halogénés, des salicylanilides halogénés, des carbanilides halogénés, des mono- et poly-alkyl- et aralkyl-phénols halogénés, et des bisphénols halogénés.

28. Etui de lentille de contact selon la revendication 27, dans lequel ledit agent antimicrobien est un diphényléther halogéné substitué par 1 à 3 groupes hydroxyles et 1 à 4 halogènes choisis dans le groupe constitué par le brome et le chlore.

29. Etui de lentille de contact selon la revendication 28, dans lequel ledit agent antimicrobien est le triclosan.

30. Etui de lentille de contact selon la revendication 24, dans lequel ledit agent antimicrobien est choisi dans le groupe constitué par le 4',5-dibromosalicylanilide ; le 3,4',5-tribromosalicylanilide ; le 3,4',5-trichloro-salicylanilide ; le 3,4,4'-trichlorocarbanilide ; le 3-trifluorométhyl-4,4'-dichlorocarbanilide ; le 2,2'-méthylènebis(3,4,6-trichlorophénol); le 5-chloro-2-(2,4-dichlorophénoxy)-phénol ; la Tyrothricine ; le chlorure de N-méthyl-N-(2-hydroxyéthyl)-N-(2-hydroxydodécyl)-N-benzylammonium ; le chlorure de cétylpyridinium.

31. Etui de lentille de contact selon la revendication 24, dans lequel ledit agent antimicrobien a une solubilité dans l'eau à 25 °C inférieure à 10 g/litre.

32. Etui de lentille de contact selon la revendication 1,
dans lequel ladite matière polymère est une polyoléfine semi-cristalline ayant une pluralité de zones amorphes ;
dans lequel ledit agent antimicrobien est un diphényléther halogéné, et
dans lequel ledit diphényléther présente une migration contrôlée à travers lesdites zones amorphes dudit corps vers la surface de celui-ci lorsqu'un déséquilibre de la pression de vapeur dudit diphényléther requiert une égalisation.

33. Etui de lentille de contact selon l'une quelconque des revendications 1 à 32, dans lequel ledit agent antimicrobien est présent à une concentration de 0,001 à 10 % en poids, de préférence de 0,1 à 0,5 %, en particulier de 0,15 à 0,25 %

34. Procédé de stockage d'une lentille de contact, comprenant les étapes consistant à :
fournir un étui de lentille de contact ayant des caractéristiques antimicrobiennes, comprenant un corps polymère définissant au moins une cavité pour maintenir ladite lentille de contact,
**caractérisé en ce qu'**un diphényléther halogéné est distribué uniformément à travers ledit corps, ledit corps polymère étant composé d'une polyoléfine semi-cristalline ayant une pluralité de zones amorphes ; et dans lequel ledit diphényléther présente une migration contrôlée à travers lesdites zones amorphes dudit corps vers la surface de celui-ci lorsqu'un déséquilibre de pression de vapeur dudit diphényléther requiert une égalisation ; et
placer ladite lentille de contact et une solution aqueuse isotonique dans ladite cavité ; et
fermer ladite cavité pour empêcher la contamination environnementale.

35. Procédé de stockage d'une lentille de contact, comprenant les étapes consistant à :
fournir un étui de lentille de contact tel que revendiqué dans la revendication 1 ;
placer ladite lentille de contact et une solution aqueuse isotonique dans ladite cavité ; et
fermer ladite cavité pour empêcher la contamination environnementale.

36. Procédé de stockage d'une lentille de contact, comprenant les étapes consistant à :
fournir un étui de lentille de contact tel que revendiqué dans la revendication 24 ;
placer ladite lentille de contact et une solution aqueuse isotonique dans ladite cavité ; et
fermer ladite cavité pour empêcher la contamination environnementale.
